# EUROPEAN PATENT SPECIFICATION

(11) **EP 0 426 455 B1**
(45) Date of publication and mention of the grant of the patent: **06.09.1995**
(21) Application number: 90311931.1
(22) Date of filing: 31.10.1990
(51) Int. Cl.: C12N 15/10, C12N 15/85, C12N 15/28

(54) **Cloning vector plasmid, vector-primer derived therefrom and preparation method of cDNA bank using the same**
Klonierungs-Plasmid-Vektor, Primer-Vektor davon und Herstellungsverfahren einer cDNA-Bank
Vecteur-plasmide de clonage, amorce de vecteur provenant de celui-ci et méthode de préparation d'une banque de données cDNA

(30) Priority: 31.10.1989 JP 283674/89; 07.09.1990 JP 238332/90
(43) Date of publication of application: 08.05.1991
(73) Proprietor: SAGAMI CHEMICAL RESEARCH CENTER, Chiyoda-ku Tokyo 100 (JP)
(72) Inventor: Kato, Seishi, Sagamihara-shi, Kanagawa 228 (JP); Aoki, Takashi, Tanashi-shi, Tokyo 188 (JP); Umezawa, Yuri, Chigasaki-shi, Kanagawa 253 (JP)
(74) Representative: Stuart, Ian Alexander

(56) References cited:
- US-A- 4 843 003
- GENE, vol. 66, 1988, pages 121-134, Amsterdam, NL; S.C. PRUITT: "Expression vectors permitting cDNA cloning and enrichment for specific sequences by hybridization/selection"
- GENE, vol. 52, 1987, pages 197-206, Amsterdam, NL; M.J. PALAZZOLO et al.: "A family of lambda phage cDNA cloning vectors, lambdaSWAJ, allowing the amplification of RNA sequences"

## Description

The present invention relates to a multi-functional cloning vector, a vector-primer derived therefrom, and a method to prepare cDNA bank using the same. This invention provides a method to obtain readily a cDNA which can be used to produce a large amount of protein which is available as a medicine.

Proteins constituting cells in our body play an central role to maintain our life as a skeleton of cell, a catalyst of reaction, and a mediator of signal transduction. Thus finding new proteins and elucidating those structure and function are important not only to know life but also to use them as a medicine or a reagent for diagnosis. Although there have been many trials to use these proteins functioning in our body as a medicine, few proteins have been available because of difficulty of preparing a large amount of protein. The recent progress of genetic engineering made it easy to find a gene encoding an useful protein from human cells and to express it in bacteria or animal cells in high yields. Using this technique recently many proteins were developed as a medicine.

Any human protein has a potential availability as a medicine because it functions in our body. It has been known that many genetic diseases are caused by disappearance of a protein playing an important role in a living cell. Therefore to produce a large amount of protein and to elucidate its function and the relationship to the disease are an important step for developing new medicine. If the investigation is begun from the purification of a protein showing a target activity, it takes a long time and much labor to obtain a large amount of starting material. To avoid this problem, the inventors take a strategy to approach from a gene by which an information of amino acid sequence of protein is encoded. In our plan, cDNA (complementary DNA) is synthesized from all mRNA which is transcribed from genome and is translated to protein, and then each cDNA is characterized on the encoding protein.

The number of genes on human genome is estimated to be approximately 10⁵, which may correspond to the number of human proteins. Recently the determination of whole sequence of human genome is discussed world-widely, which is called the Human Genome Project. If this plan is realized, the amino acid sequence of all proteins is expected to be elucidated. It is, however, difficult to determine the amino acid sequence only from the nucleotide sequence of genome because the genome consists of exons encoding a part of protein and introns not encoding protein. In the cell, mRNA transcribed from the genome is processed by deletion of intron and the processed mRNA containing only exons is transcribed to protein on ribosome. Therefore if we determine the nucleotide sequence of the processed mRNA, we can obtain the information of the amino acid sequence of protein encoded by mRNA. The progress of the gene recombination technique enabled us to convert mRNA to cDNA and to introduce it into *E.coli*. According to this technique we can synthesize a "cDNA vector" which contains cDNA derived from one species of mRNA, and construct the "cDNA library" which is a group of *E.coli* containing cDNA vectors. If we determine the nucleotide sequence of all cDNAs contained in the cDNA library, which corresponds to the nucleotide sequences of all mRNA existing in a cell, we obtain the amino acid sequence of all proteins synthesized in a cell. We call the group of sequence-determined CDNAs the "cDNA bank". If the obtained cDNA vector is introduced and expressed in adequate animal cells, the expression product may be used for an assay screening. Taking above strategy we can construct the cDNA bank of human proteins, which is called the "homo-protein" cDNA bank, in which the amino acid sequence of each cDNA has been determined and each cDNA can be expressed in mammalian cells. Here we describe only the case of human protein as an example, but this strategy can apply to the case of the other animal and plant cells, and we can obtain also an useful information about proteins of these cells.

The success of above strategy depends on the quality of cDNA library. From the point of this view, there are many problems in the cDNA library prepared by known methods, for example, that subcloning of a cDNA fragment to an adequate vector is required to sequence the cDNA, to screen a full-length cDNA using it as a probe, or to express it in animal cells. It takes a long time and much labor to analyze many cDNA clones by this subcloning procedure. This problem is solved by developing a novel method by which above all procedure can be carried out on the same vector. The minimum requirements for a cDNA vector satisfying above method is , 1) to contain a directional full-length cDNA clone, 2) to be sequenced easily, 3) to be used to prepare a probe which can be used for various screening, 4) to be expressed in *in vivo* or *in vitro* systems. The term "full-length cDNA clone" described in the requirement 1) means the cDNA clone containing at least whole coding region of protein encoded by mRNA in this specification, but strictly the cDNA clone containing the whole sequence of a template mRNA from 5′ end to poly A tail.

The most popular method to prepare cDNA at present is so called the Gubler-Hoffman method described below [Gene 25:263-269 (1983)]. The first strand of cDNA is synthesized from poly(A)⁺RNA template isolated from cells and an oligo dT primer using reverse transcriptase. Then the RNA strand is replaced by the second DNA strand using *E.coli* RNase H, *E.coli* DNA polymerase I, and *E.coli* DNA ligase. After blunting both ends of double-stranded cDNA by T4 DNA polymerase, an adequate oligonucleotide linker DNA is added. Then the resulting cDNA is ligated to a phage vector or a plasmid vector, and used to transform *E.coli*. It is difficult to obtain a full-length cDNA using this method, because when synthesizing a second strand 5′ terminal sequence of mRNA used as a primer is deleted and both terminals of cDNA are often deleted by exonuclease activity of DNA polymerase I or T4 DNA polymerase. The vector containing an origin and a promoter for expression in mammalian cells, a replication origin of a single-stranded phage, and an RNA polymerase promoter, for example, such as pCDM8 [B.Seed, Nature 329:840-842(1987)] is known. Using this vector, preparation of single-stranded DNA for sequencing, preparation of RNA probe, synthesis of mRNA for *in vitro* or *in vivo* translation, and expression in mammalian cell are possible, but the cDNA insertion is not directional. Thus the cDNA library prepared using the Gubler-Hoffman method dose not satisfy the requirement 1). Furthermore a large size of pCDM8 which is 4.8 kbp long is not suitable to clone a long size of cDNA.

The Okayama-Berg method [Mol.Cell.Biol. 2:161-170(1982)] is known as a method giving full-length cDNA clone at high frequency, in which the dT-tailed vector-primer is used for synthesis of the first strand cDNA from poly(A)⁺RNA by reverse transcriptase. Following addition of dC tail, the dG-tailed linker DNA is ligated and then RNA is replaced to DNA by *E.coli* RNaseH, *E.coli* DNA polymerase I, and *E.coli* DNA ligase. Since the dC tail addition occurs rarely at the 3' end of first strand cDNA extended incompletely, the dC-tailed clone gives a full-length cDNA preferentially. Using a vector-primer furthermore causes the directional insertion of cDNA. Okayama et al. [Mol.Cell.Biol. 3:280-289(1983)] have developed the expression system in mammalian cells by using linker DNA containing an origin and a promoter of SV40. Honjo has developed the expression system in *Xenopus* oocyte of mRNA transcribed *in vitro* by using linker DNA containing SP6 promoter [Japanese Patent 62-74291; EP-A-0,218,432]. However the use of Okayama-Berg method is limited because of requiring a high quality of technique. The large number of dG tails at the 5' terminus make it often difficult to sequence from 5' terminus. It is further difficult to prepare probes by this method. Thus the cDNA library using this method dose not satisfy the requirements 2) and 3) described above.

Although there have been several improved methods, no methods satisfy all of four requirements described above. Since even the lack of one requirement makes it difficult to carry out above strategy, the development of method satisfying all above requirements is necessary.

In view of the above, it is desirable to provide a method to construct a cDNA vector which satisfies the following four requirements, 1) to contain the directional full-length cDNA clone, 2) to be easily sequenced, 3) to be used to prepare an RNA probe which is complementary to a full-length cDNA and is used for screening, 4) to be able to express in a mammalian cell. A plasmid vector and a vector-primer derived from it, are desirable, for carrying out the above method.

The inventors succeeded in constructing a novel vector for cDNA cloning and in constructing a human cDNA bank using it. The invention provides a cloning vector which comprises a promoter PR1 acting in mammalian cells, followed by an unique restriction enzyme site RE1 rarely existing in eukaryotic DNA and a promoter PR2 of RNA polymerase, followed by an unique restriction enzyme site RE2 generating 3'-protruding dN tail, followed by an unique arbitrary restriction enzyme site RE3, followed by an unique arbitrary restriction enzyme site RE4 generating 3'-protruding terminus, followed by an unique restriction enzyme site RE5 rarely existing in eukaryotic DNA and a promoter PR3 of RNA polymerase, and at least one kind of arbitrary restriction enzyme site RE6 generating 3'-protruding terminus existing at the arbitrary position before the restriction site RE1, and further, at the arbitrary position except above regions, an origin OR1 for replication in mammalian cells, an origin OR2 for replication of a single-stranded phage, an origin OR3 for replication in *E.coli*, and a selective marker.

The invention further provides a vector-primer which is prepared from a cloning vector plasmid described above. After cutting a cloning vector at a restriction site RE4, the dT-tail is added by terminal deoxynucleotidyl transferase and then cut at the restriction site RE3.

The invention further provides a method to prepare a cDNA bank comprising cDNA vectors which each contain a sequence-determined cDNA insert and can be expressed in mammalian cells. This method includes synthesizing cDNAs from poly(A)⁺RNA isolated from cell using vector-primer described above, transforming *E.coli* with them, preparing a cDNA plaque library by infection of a helper phage, and sequencing cDNA from 5' terminal end using a single-stranded DNA isolated from a single-stranded phage plaque.

The invention further provides a method to prepare a library-specific cDNA bank including cDNA vectors which each contain a sequence-determined cDNA insert and can be expressed in mammalian cells. This method comprises preparing two kinds of cDNA library LA and LB using above method, preparing sense RNA probes from the library LA using RNA polymerase, preparing a group of anti-sense single-stranded cDNAs from the library LB, obtaining the library LA - specific cDNA clones by screening the library LA using probes which contains a hybridization mixture between RNA probes and single-stranded cDNAs prepared above, and sequencing them from 5′ terminus.

Using techniques embodying the invention we may clone a full-length cDNA without a complicated procedure such as subcloning, determine its nucleotide sequence, prepare a complementary probe for screening, and express it in mammalian cells. Therefore this cloning vector is a powerful tool especially to prepare a cDNA bank originated from various mRNAs. The "cDNA bank" in this specification means the cDNA library in which the nucleotide sequence of each cDNA clone has been already determined. It may be possible to determine the amino acid sequence of all proteins composed of human or any other animals' cell in shorter period than using conventional methods.

### BRIEF DESCRIPTION OF THE DRAWINGS

Figure 1 shows the functions of a cDNA vector according to an embodiment of this invention;
Figure 2 shows the arrangement of promoters in a cloning vector according to an embodiment of this invention;
Figure 3 shows the restriction map of the vector pKA1;
Figure 4 shows a process to synthesize cDNA using a vector plasmid;
Figure 5 shows the subtraction method using a cDNA system according to an embodiment of this invention;
Figures 6 to 8 show the procedures to construct vectors according to embodiments of this invention;
Figure 9 shows the method to prepare a vector-primer according to an embodiment of this invention;
Figure 10 shows the results of plaque hybridization and subtraction using the protocol according to an embodiment of this invention.

### DETAILED DESCRIPTION

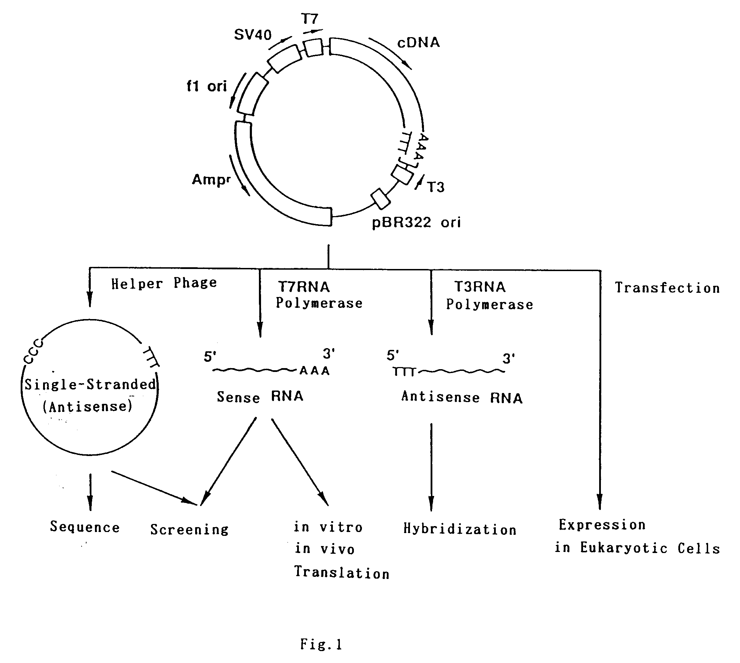

Figure 1 shows the functions of a cDNA vector embodying the invention. The construction method of a cloning vector, the preparation method of a cDNA vector using it, and examples showing that this cDNA vector satisfies above four requirements will now be described.

### (1) Cloning Vector

The most characteristic point in this invention is the cloning vector used for cDNA synthesis. The vector-primer contains the full-length cDNA clone and is preferably endowed with the multi-functional properties to satisfy the requirements 2), 3) and 4). As described above, the cloning vector included in this invention comprises a promoter PR1 acting in mammalian cells, followed by an unique restriction enzyme site RE1 rarely existing in eukaryotic DNA and a promoter PR2 of RNA polymerase, followed by an unique restriction enzyme site RE2 generating 3′-protruding dG tail, followed by an unique arbitrary restriction enzyme site RE3, followed by an unique arbitrary restriction enzyme site RE4 generating 3′-protruding terminus, followed by an unique restriction enzyme site RE5 rarely existing in eukaryotic DNA and a promoter PR3 of RNA polymerase, and at least one kind of arbitrary restriction enzyme site RE6 generating 3′-protruding terminus existing at the arbitrary position before the restriction site RE1, and further, at the arbitrary position except above region, an origin OR1 for replication in mammalian cells, an origin OR2 for replication of a single-stranded phage, an origin OR3 for replication in E.coli, and a selectable marker.

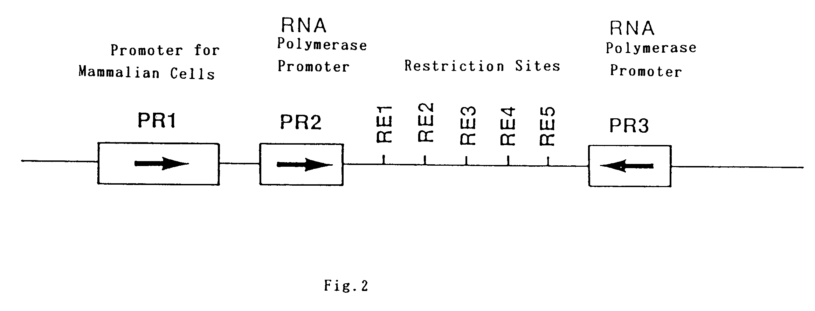

The term " unique" in the above description means that the restriction site appears only once on the plasmid. Figure 2 shows the arrangement of promoters PR1, PR2, and PR3. The change of the order of RE1 and PR2 as well as the order of RE5 and PR3 is allowable. It is desirable that no extra nucleotide sequence exists between the restriction site and promoter. The restriction sites RE2, RE3, and RE4 are necessary to satisfy the requirement 1), OR2 and RE6 to satisfy the requirement 2), PR2, PR3, RE1, RE5, and OR2 to satisfy the requirement 3), and OR1 and PR1 to satisfy the requirement 4).

A promoter for expression in mammalian cells, PR1, includes promoters such as an early or late promoter of SV40, a late promoter of adenovirus 2, and a 5′ long terminal repeat (LTR) of retrovirus. An unique restriction enzyme site rarely existing in eukaryotic DNA, RE1 and RE5, includes restriction sites such as NotI, SnaBI, SfiI, and SpeI. An unique restriction enzyme site producing 3′-protruding dG oligomer tails after digestion, RE2, includes restriction sites such as BstXI, SfiI, DraIII, and BglI. Since the nucleotide sequence of cut site in these restriction enzyme sites can be replaced by any nucleotide pairs, if dG-dC pair as an nucleotide pair is used in them, cutting by these restriction enzymes produces a 3′-protruding dG terminus. For example, the nucleotide sequence of restriction site for BstXI is shown as below:
5′-CCANNNNNNTGG-3′
3′-GGTNNNNNNACC-5′
If replacing N in the top sequence by G, digestion of this site with BstXI produces the following terminal sequence.
5′-CCAGGGGG-3′
3′-GGTC-5′
Thus RE2 includes any kind of restriction sites which can produce a 3′-protruding terminus after restriction enzyme digestion, and so is not limited by the above examples.

RE6, which comprises at least one kind of arbitrary restriction enzyme site producing 3′-protruding terminal existing at the arbitrary position before the restriction site RE1, includes any restriction site producing a 3′-protruding terminus as far as it is different from RE1, RE2, RE3, RE4, and RE5, and preferably exists rarely in eukaryotic DNA. One example is SfiI, but there are many other examples. It is desirable that RE6 contain more than two different restriction sites. It is not necessary that RE6 is an unique site but that exists at the upstream of RE1.

PR2 and PR3 for an RNA polymerase promoter includes promoters of polymerases such as T3 RNA polymerase, T7 RNA polymerase, and SP6 RNA polymerase. OR1 for a replication origin in mammalian cells includes an origin of SV40, for example. OR2 for a replication origin of a single-stranded phage includes origins of f1 phage or M13 phage. OR3 for a replication origin of *E.coli* includes origins of plasmids such as pBR322 or pUC series. A selective marker includes drug-resistant markers such as those for ampicillin, tetracyclin, or kanamycin, and the other markers such as nutrient requirement or temperature sensitivity. Although a neomycin-resistant gene can be introduced to this vector as a drug-resistant marker for a mammalian cell system, this may cause a disadvantage that the size of vector becomes too long. It is further desirable to include a splicing junction and poly A addition signal sequence for high expression in mammalian cells.

A cDNA is introduced in the directional manner between the restriction sites RE2 and RE4, in which 5′-terminal of mRNA is adjacent to RE2. The direction of a replication origin of a single-stranded phage determines which strand of double strands in vector plasmid is produced as a single strand. In this vector system, the single-stranded DNA consists of an anti-sense strand for cDNA. In this specification, the strand possessing the same sequence as template mRNA except U replaced by T is defined as a sense strand, and the complementary sequence as an anti-sense strand.

To determine the nucleotide sequence from 5′ terminal of cDNA, any synthetic oligonucleotide which anneals at the upstream of RE2 can be used as a sequencing primer. If the vector is designed to have the nucleotide sequences of primers used for sequencing of M13, these primers can be used for sequencing of a cDNA insert.

An RNA polymerase promoter PR2 is introduced into the vector in the direction as producing a single-stranded RNA of sense strand for mRNA template. In the same manner, PR3 is introduced to produce an anti-sense strand. The plasmid described above can be readily constructed using known plasmids and synthetic oligonucleotides. The plasmid pKA1 described below as an example uses an early promoter of SV40 for PR1, SnaBI for RE1, T7 promoter for PR2, BstXI for RE2, EcoRV for RE3, KpnI for RE4, NotI for RE5, a T3 promoter for PR3, SfiI, PstI, and SphI for RE6, an origin of SV40 for OR1, an origin of phage f1 for OR2, an origin of pBR322 for OR3, an ampicillin resistant marker (β-lactamase gene) for a drug-resistant marker, and the nucleotide sequences of universal and reverse primer of M13 phage for the priming site where a sequencing primer anneals, respectively. pKA1 further includes a 16S splicing site at the downstream of a SV40 promoter and a poly A addition signal sequence at the downstream of a T3 promoter. The size of pKA1 is 3.6 kbp and is shorter than known multi-functional cloning vectors, that makes it easy to get longer cDNA clones.

The plasmid described above is cut at the restriction site RE4 to produce a 3′-protruding terminal, and then dT tail is added to the terminal using terminal deoxynucleotityl transferase. The desirable number of added dT is from 30 to 70, preferably from 50 to 60. Cutting at the restriction site RE3, the longer DNA fragment containing a vector is isolated with agarose gel electrophoresis and is used as a vector-primer.

### (2) Preparation method of cDNA library

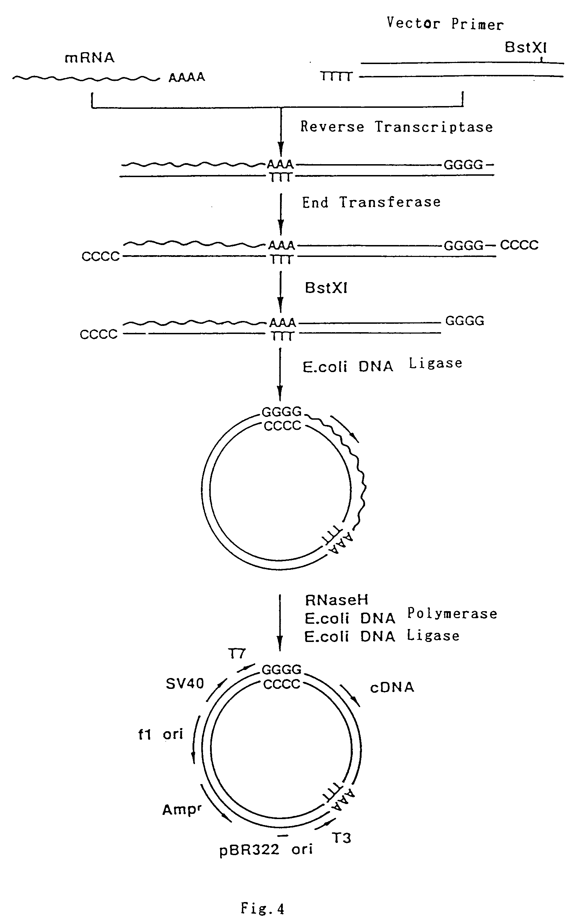

Poly(A)⁺RNA isolated from cells using a known method is annealed with a vector-primer described above, and then reverse transcriptase is used to synthesize the first strand of cDNA. The dC tail, the number of which is preferably from 4 to 10, is added to the end of the first strand cDNA using terminal deoxynucleotidyl transferase. After cutting at the restriction site RE2, self-ligation is carried out using *E.coli* DNA ligase. Then the RNA strand is replaced by the second cDNA strand using *E.coli* RNase H, *E.coli* DNA polymerase I, and *E.coli* DNA ligase. This procedure is shown in Figure 4. The obtained ligation mixture containing cDNA-harboring plasmid is used to transform a host *E.coli* containing a F′ factor. An *E.coli* strain containing a F′ factor includes hosts such as NM522, JM101, JM103, JM105, JM109, and DH5αF′. Transformants constituting a cDNA library are cultured and amplified in an appropriate medium such as L-broth or 2xTY containing ampicillin if the vector has an ampicillin-resistant gene, and then are stocked in a deep freezer after adding glycerol or DMSO. If the culture of cDNA library is infected with a helper phage, each *E.coli* cell releases in a medium a phage containing a single-stranded cDNA derived from a cDNA vector. This single-stranded cDNA have an anti-sense strand for mRNA template. After infection of helper phage to cDNA library and incubation, E.coli cell is harvested, washed thoroughly with medium to remove helper phage, and diluted with medium. This diluted cell solution is mixed with a soft agar or a soft agarose solution containing *E.coli* host not harboring cDNA vector, the mixture is poured onto agar plates. After incubation plaques can be seen where cDNA-harboring cells exist. *E.coli* cells picked up from a single plaque by a toothpick are cultured in a liquid medium such as 2xTY. A part of culture medium is stored in a freezer. After the remaining culture is centrifuged, the cell pellet is used to isolate plasmid and the supernatant is used to isolate phage from which a single-stranded cDNA is isolated by known methods. It is desirable that a part of supernatant containing phage is stored in a refrigerator. A helper phage used above includes phages such as M13KO7 [J.Vieira and J.Messing, " Methods in Enzymology" vol.153, p.3, 1987], VCS-M13, and R408 (both can be purchased from Stratagene).

We can select an alternative procedure to isolate a single-stranded DNA, in which before phage infection a single colony is picked up, incubated, and followed by helper phage infection.

Using above method, various types of cDNA libraries can be prepared, such as transformants containing cDNA vectors originated from a single species of mRNA, cDNA vectors as a double-stranded DNA, cDNA vectors as a single-stranded DNA, and phages containing a single-stranded DNA.

### (3) Determination method of nucleotide sequence

Either a single-stranded DNA or a double-stranded DNA can be used. A single-stranded DNA is preferred because more nucleotide sequences can be read. The nucleotide sequence can be determined using a dideoxy method in which synthetic oligonucleotide of 17 mer to 20 mer corresponding to the nucleotide sequence just before the restriction site RE2 is used as a sequencing primer and substrates labeled with radioisotope or fluorescent dye are used. A sequencing reaction is carried out using enzymes such as Klenow fragment, Taq DNA polymerase, T7 DNA polymerase, and reverse transcriptase. Using pKA1, an universal primer can be used for M13 sequencing, by which approximately 400 nucleotides from 5′-terminal of cDNA can be determined. If cDNA is a full-length clone, an initiation codon followed by an open reading frame in the determined sequence may be found. If no stop codon appears within the open reading frame, it is necessary to determine further sequence, in which case new primer corresponding to the nucleotide sequence near the 3′-end of the determined sequence should be synthesized to be used for further sequencing. Using the plasmid of the present invention, the derivatives deleted from a 5′ terminal of a cDNA insert can be prepared by cutting the cDNA-harboring plasmid at restriction sites RE1 and RE6 and followed by exonuclease treatment according to the known deletion method. These derivatives can be used to determine the nucleotide sequence using a primer corresponding to the nucleotide sequence just before the restriction site RE6, which corresponds to the reverse primer for M13 in the case of pKA1 system.

Since the obtained nucleotide sequence corresponds to the sequence of a sense strand which is the same as the sequence of mRNA, this sequence can be used to search similarity to known sequences in database: a DNA database for a nucleotide sequence and a protein database for an amino acid sequence of an open reading frame. If there is similarity to a known sequence, the function and characteristics of the protein encoded by the obtained cDNA can be estimated.

### (4) Screening Method

When determination of sequences of all cDNA clones included in the library is desired, it is desirable to remove the already sequenced clones from the library to avoid the overlapped sequencing of the same clone. One aspect of the invention provides a subtraction method satisfying above demand. This method also can be used to obtain cell-specific clones or stimulus-induced clones.

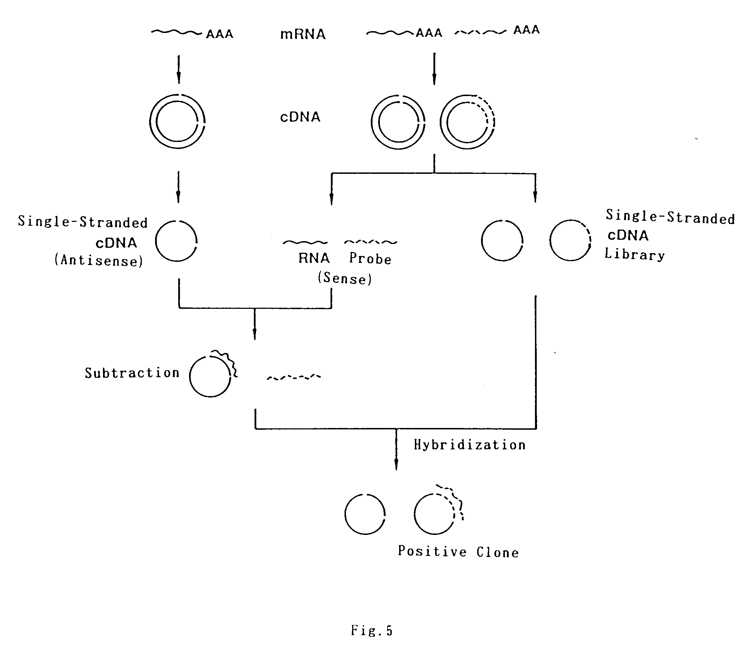

An example that a cDNA library LA is subtracted by a cDNA library LB will now be described. This case corresponds that a library LA contains novel clones or specific clones, on the other hand, a library LB contains sequence-determined clones or common clones. The library LA is cultured and amplified, and total plasmids containing cDNA vectors are isolated, which are cut at the restriction site RE5 and then reacted with the RNA polymerase acting on a promoter site PR2. If the substrate nucleotide labeled with radioisotope or fluorescence dye is added in the reaction mixture, the labeled RNA corresponding to the sense strand for each cDNA is obtained. On the other hand, the library LB is cultured and infected by a helper phage to prepare a single-stranded DNA from the supernatant. When the labeled RNAs prepared from library LA and the single-stranded DNA prepared from library LB are mixed and hybridized, the sense RNA strand hybridizes with the corresponding anti-sense single-stranded DNA derived from the common cDNA clones existing in both libraries, but labeled RNAs derived from specific cDNA clones in a library LA remains to be free. If these hybridization mixture is used as a probe to screen a library LA by the methods of plaque hybridization or colony hybridization, specific clones existing only in a library LA can be obtained. The principle of the subtraction method described above is shown in Figure 5.

Since the cDNA vector described in this invention has a promoter PR3 for an RNA polymerase after the 3′ terminal of cDNA, an RNA probe which consists of an anti-sense strand for cDNA can be prepared by cutting the cDNA vector at the restriction site RE1 and reacting with an RNA polymerase acting on PR3. The product can be used as a probe for Northern hybridization.

### (5) Expression in animal cells

Since the cDNA vector described in this invention has a replication origin and promoter functioning in mammalian cells, this can be used to express in mammalian cells if the isolated CDNA-harboring plasmid is introduced into cells by using known methods such as a phosphate calcium method or an electroporation method. If the introduced cDNA encodes a receptor protein or a secreted protein, this method can be used for an expression screening.

Cutting at the restriction site RE5 and reacting it with CAP-like nucleotide substrate such as m⁷GpppG and RNA polymerase acting on PR2, RNA which is available in in vitro or Xenopus oocyte translation systems can be obtained.

The present invention will now be described by way of examples, but various changes and modifications can be made without departing from the invention.

The basic procedure of DNA recombination and the reaction conditions were in accordance with the literature [T.Maniatis et al. (1982), "Molecular Cloning. A Laboratory Manual", Cold Spring Harbor Laboratory].

### Example 1

### Construction of Cloning Vector pKA1

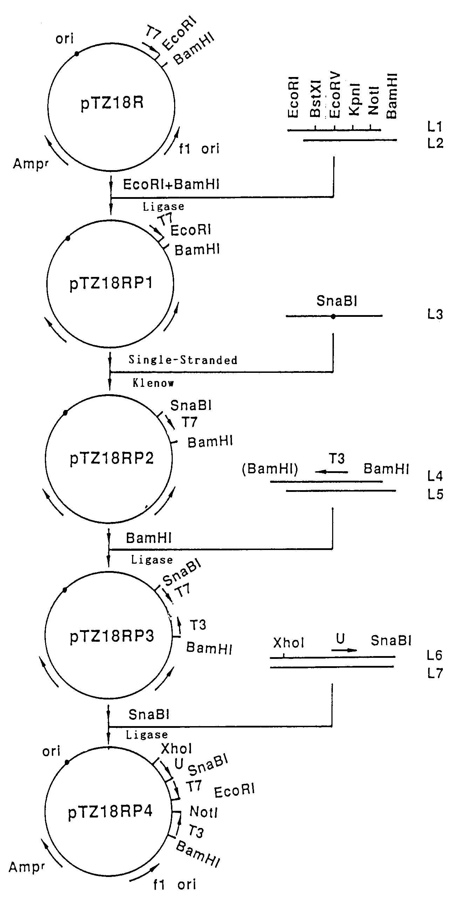

### (1) Preparation of pTZ18RP1 (Figure 6)

Ten µg of the plasmid pTZ18RP1 purchased from Pharmacia was digested by 100 units of BamHI and 100 units of EcoRI. The reaction mixture was fractionated with 0.8% agarose gel electrophoresis (AGE), and then a DNA fragment of 2.9 kbp was isolated from the gel. Ten pmoles of synthetic oligomers L1 and L2, which contain the restriction enzyme sites EcoRI, BStXI, EcoRV, KpnI, NotI, and BamHI, were annealed and ligated to above DNA fragment. 
L1     5'-AATTCCAGGGGGGTGGTGGATATCCTGGTACCGCGGCCGCG-3'

L2         3'-GGTCCCCCCACCACCTATAGGACCATGGCGCCGGCGCCTAG-5'
E.coli HB101 was transformed with the ligation mixture and plasmids were isolated from several transformants. After the insertion site of synthetic oligomer in plasmids was sequenced using a reverse primer for M13 sequencing, the plasmid possessing the designed sequence was denoted by pTZ18RP1.

### (2) Preparation of pTZ18RP2 (Figure 6)

The SnaBI site was introduced before a T7 promoter using site-directed mutagenesis. Transformant of *E.coli* CJ236 (purchased from BioRad) by the plasmid pTZ18RP1 prepared in section (1) was cultured in 5 ml of 2xTY medium containing 50 µg/ml of ampicillin at 37 °C for one hour. Then a helper phage M13KO7 (purchased from Pharmacia) solution was added and inoculated at 37 °C overnight. A single-stranded DNA (ssDNA) was isolated from the culture medium. The ssDNA annealed with 10 pmoles of synthetic oligomer L3 in which 5′ terminal was phosphorylated by T4 polynucleotide kinase was mixed with 4 units of Klenow fragment, 350 units of T4 DNA ligase, and 0.8 mM of dNTPs followed by incubation at 4 °C for 5 minutes, at room temperature for 5 minutes, and finally at 37 °C for 2 hours.
L3 5′-CCATGATTACGTATTTAATACGA-3′
*E.coli* JM109 was transformed with the ligation mixture and plasmids were isolated from several transformants. The plasmid which can be cut by SnaBI and possesses the designed sequence was denoted by pTZ18RP2.

### (3) Preparation of pTZ18RP3 (Figure 6)

A T3 promoter was introduced to the BamHI site in pTZ18RP2. Ten µg of pTZ18RP2 prepared in section (2) was digested by 100 units of BamHI. After the reaction mixture was fractionated with 0.8% agarose gel electrophoresis, a DNA fragment was isolated from the gel. Ten pmoles of synthetic oligomers L4 and L5 containing a T3 promoter were annealed and ligated to above DNA fragment. 
L4  5'-GATCTCCCTTTAGTGAGGGTTATTG-3'
L5      3'-AGGGAAATCACTCCCAATAACCTAG-5'
*E.coli* NM522 was transformed with the ligation mixture and plasmids were isolated from several transformants. The plasmid possessing the designed sequence was denoted by pTZ18RP3.

### (4) Preparation of pTZ18RP4 (Figure 6)

The XhoI site and universal primer sequence (designated by U) for M13 sequencing was introduced to the SnaBI site in pTZ18RP3. Ten µg of pTZ18RP3 prepared in section (3) was digested by 100 units of SnaBI. After the reaction mixture was fractionated with 0.8% agarose gel electrophoresis, a DNA fragment was isolated from the gel. Ten pmoles of synthetic oligomers L6 and L7 containing a XhoI site and universal primer sequence (designated by U) for M13 sequencing were annealed and ligated to above DNA fragment. 
L6  5'-GCCTCGAGTGTAAAACGACGGCCAGTAC- 3'

L7  3'-CGGAGCTCACATTTTGCTGCCGGTCATG- 5'
*E.coli* NM522 (purchased from Stratagene) was transformed with the ligation mixture and plasmids were isolated from several transformants. The plasmid possessing the designed sequence was denoted by pTZ18RP4.

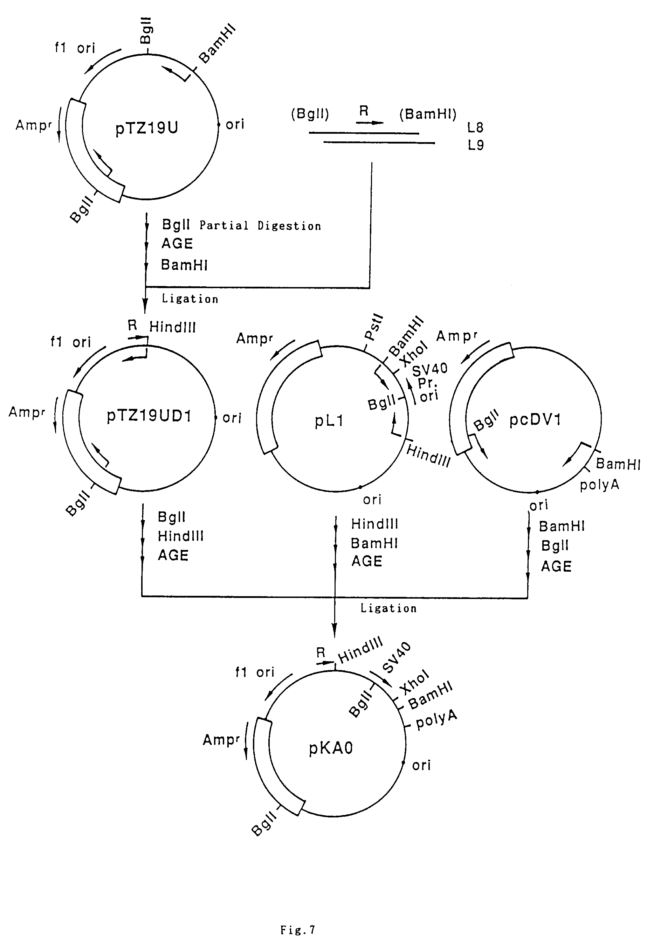

### (5) Preparation of pTZ19UD1 (Figure 7)

A reverse primer sequence (designated by R) for M13 sequencing was introduced between BglI and BamHI sites in the plasmid pTZ19U. Two µg of pTZ19U (purchased from Toyobo) was partially digested by 20 units of BglI at 37 °C for 15 minutes. A fragment of 2.8 kbp cut at one site was isolated from the gel after fractionated with 1.2% agarose gel electrophoresis. After this fragment was digested by 100 units of BamHI, a fragment of 2.7 kbp was isolated with 1.2% agarose gel electrophoresis. Ten pmoles of synthetic oligomers L8 and L9 containing a reverse primer sequence (designated by R) for M13 sequencing were annealed and ligated to the BamHI-BglI fragment of pTZ19U. 
L8 5'-ACACAGGAAACAGCTATGACCAT-3'
L9 3'-AAGTGTGTCCTTTGTCGATACTGGTACTAG-5'
*E.coli* JM109 was transformed with the ligation mixture and plasmids were isolated from several transformants. The plasmid possessing the designed sequence was denoted by pTZ19UD1.

### (6) Preparation of pKA0 (Figure 7)

Ten µg of pTZ19UD1 prepared in section (5) was digested by 100 units of BglI and 100 units of HindIII. After fractionated with 1.2 % agarose gel electrophoresis, a 1.4 kbp fragment containing a f1 origin was isolated from the gel. Ten µg of pL1 (purchased from Pharmacia) was digested by 100 units of HindIII and 100 units of BamHI. After fractionated with 1.8 % agarose gel electrophoresis, a 420 bp fragment containing an origin and early promoter of SV40 was isolated from the gel. Ten µg of pcDV1 (purchased from Pharmacia) was digested by 100 units of BamHI and 100 units of BglI. After fractionated with 0.8 % agarose gel electrophoresis, a 1.6 kbp fragment containing a poly A addition signal sequence was isolated from the gel. These three fragments were ligated and then *E.coli* HB101 was transformed with the ligation mixture. Plasmids were isolated from several transformants and digested by BamHI, BglI and HindIII to confirm that the restriction map of the obtained plasmid agrees with that of pKA0.

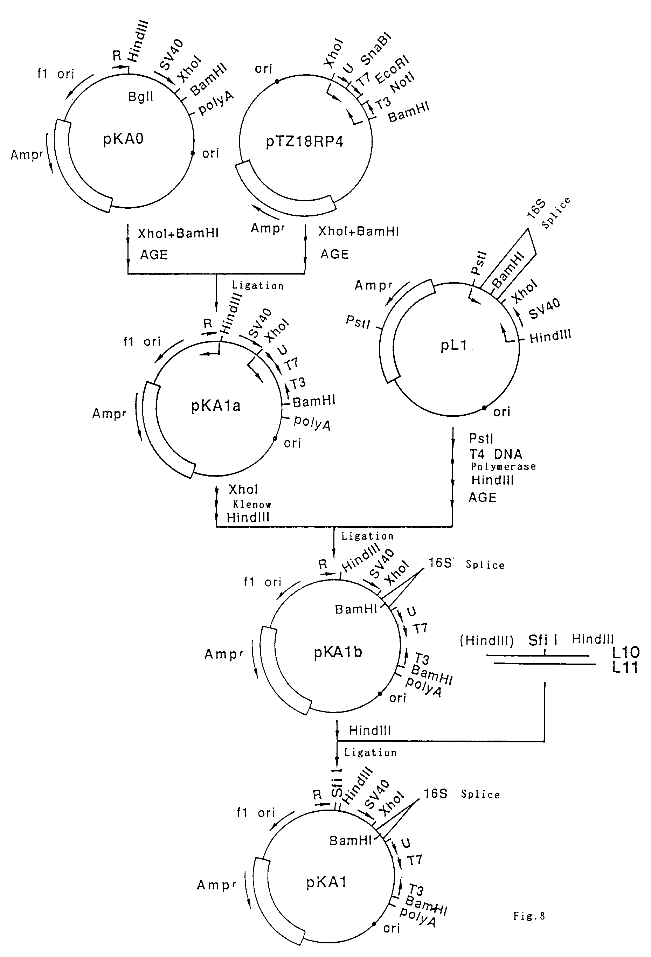

### (7) Preparation of pKA1a (Figure 8)

After 10 µg of pTZ18RP4 prepared in section (4) was digested by 100 units of BamHI and 100 units of XhoI, a fragment of 117 bp was isolated with 1.8 % agarose gel electrophoresis. On the other hand, after 10 µg of pKA0 prepared in section (6) was digested by 100 units of BamHI and 100 units of XhoI, a fragment of 3.3 kbp was isolated with 0.8 % agarose gel electrophoresis. Both fragments were ligated and then *E.coli* HB101 was transformed with the ligation mixture. Plasmids were isolated from several transformants and digested by BamHI and XhoI to confirm that the restriction map of the obtained plasmid agrees with that of pKA1a.

### (8) Preparation of pKA1b (Figure 8)

Ten µg of pL1 was digested by 100 units of PstI, and the terminals were blunted by T4 DNA polymerase. After this fragment was digested by HindIII, a 500 bp fragment containing a SV40 promoter and 16S splicing site was isolated with 1.8% agarose gel electrophoresis.

One µg of PkA1a was digested by 100 units of XhoI, and the terminals were blunted by Klenow fragment. After this fragment was digested by HindIII, a fragment of 3 kbp was isolated with 0.8% agarose gel electrophoresis.

Both fragments were ligated and then *E.coli* HB101 was transformed with the ligation mixture. Plasmids were isolated from several transformants and digested by HindIII and BamHI to confirm that the restriction map of the obtained plasmid agrees with that of pKA1b.

### (9) Preparation of pKA1 (Figure 8)

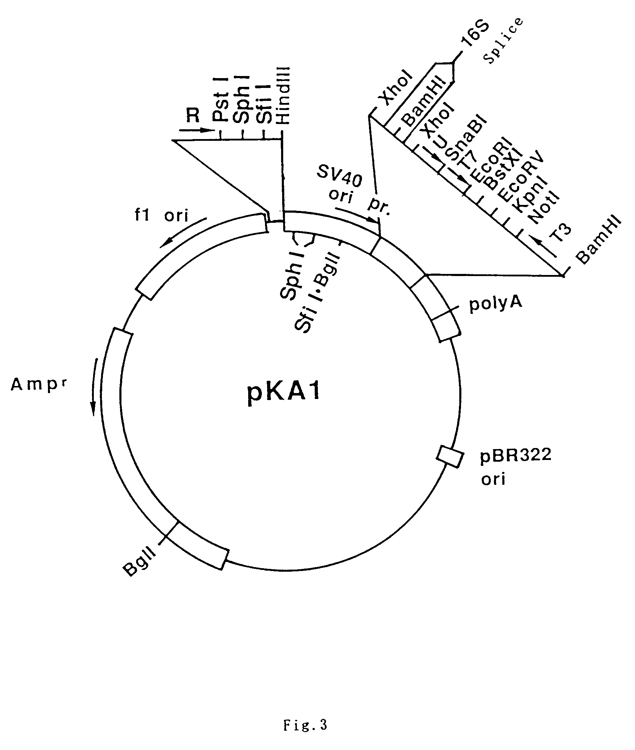

After 10 µg of pKA1b was digested by 100 units of HindIII, a fragment of 3.6 kbp was isolated with 1.8 % agarose gel electrophoresis. Ten pmoles of synthetic oligomers L10 and L11 containing a restriction site SfiI were annealed and ligated to the above fragment. 
L10 5'-AGCTAGGCCTACATGGCCA-3'
L11 3'-TCCGGATGTACCGGTTCGA-5'
*E.coli* HB101 was transformed with the ligation mixture and plasmids were isolated from several transformants. After the insertion site of oligomers was sequenced using a reverse primer for M13 sequencing, the plasmid possessing the designed sequence, in which the order of restriction sites is SphI-SfiI-HindIII, was denoted by pKA1. The details of restriction map of pKA1 has been already shown in Figure 3.

### EXAMPLE 2

### Preparation OF cDNA Library

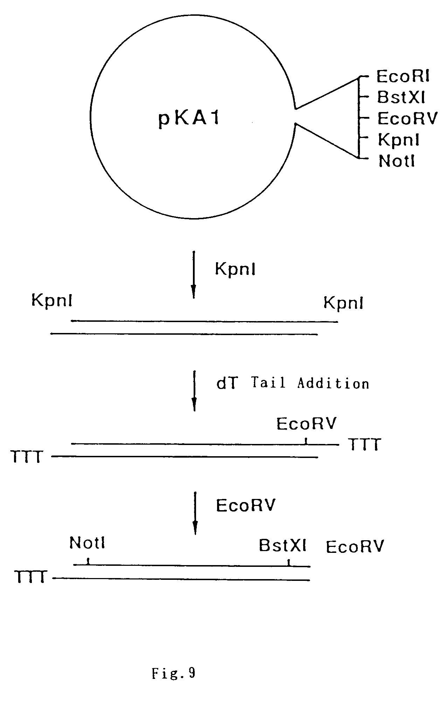

### (1) Preparation of vector-primer (Figure 9)

After 100 µg of pKA1 was digested by 200 units of KpnI, a fragment was isolated with 1.8 % agarose gel electrophoresis. A fragment of 70 µg was incubated at 37 °C for 30 minutes in the reaction mixture containing 20 µM of dTTP and 375 units of terminal deoxynucleotidyl transferase (purchased from Takara Shuzo). The number of tailed dT was estimated to be approximately 60, which was calculated from the uptake of [α-³²P]dTTP added to a part of the reaction mixture. After digested with EcoRV and fractionated with 0.8% agarose gel electrophoresis, a longer fragment was isolated and used as a vector-primer.

### (2) Preparation of poly(A)⁺RNA

Total mRNA was isolated using a guanidinium/thiocyanate method from the cell culture of human cell lines such as HUT-78, HUT-102, U937, and HT-1080. Poly(A)⁺RNA was purified from this mRNA using an oligo dT column.

### (3) Synthesis of cDNA

Total process of cDNA synthesis is shown in Figure 4. The reaction condition described in the protocol of the Okayama-Berg method was used. Enzymes were purchased from Takara Shuzo. Briefly, after annealing 3 µg of poly(A)⁺RNA prepared in section (2) with 1.5 µg of vector-primer prepared in section (1), reverse transcriptase was added to synthesize the first cDNA strand. The reaction mixture was extracted with phenol, and then cDNA was precipitated by adding ethanol. The precipitate was dissolved in the reaction solution containing 1 µM of dCTP and reacted with 15 units of terminal deoxynucleotidyl transferase to add a dC tail. After phenol extraction and ethanol precipitation of the reaction mixture, the product was digested with 50 units of BstXI (purchased from New England Biolabs). After phenol extraction and ethanol precipitation of the reaction mixture, the product was annealed and self-ligated using *E.coli* DNA ligase. Then adding dNTPs including dATP, dGTP, dCTP, and dTTP, *E.coli* DNA polymerase I and *E.coli* RNase H, the RNA strands were replaced by the DNA strands.

### (4) Preparation of cDNA library

*E.coli* NM522 was transformed with the solution containing cDNA vectors prepared in section (3). Transformants were suspended in 2xTY medium containing 100 µg/ml of ampicillin and incubated at 37 °C overnight. A part of transformant was spread on an agar plate and incubated overnight. The number of independent clones including in the library was estimated by counting the number of colonies appeared on the agar plate to be approximately 2 x 10⁵ ∼ 10⁶. A part of culture was infected by helper phage M13KO7 (purchased from Pharmacia) and incubated at 37 °C overnight after adding kanamycin to 70 µg/ml. A part of the resulting culture was stocked at -80 °C as a 15% glycerol solution.

### (5) Isolation of cDNA vector plasmid

The *E.coli* culture consisting of the cDNA library prepared in section (4) was harvested by centrifugation. The resulting pellet was washed thoroughly with 2xTY medium, mixed with a culture of *E.coli* NM522 and a soft agar medium kept warm at 37 °C, and poured onto 2 x TY agar plates. After incubation at 37 °C overnight, a single plaque appeared on plates was isolated, suspended in 2 x TY medium, and inoculated at 37 °C overnight. The culture was separated to the cell pellet and the supernatant by centrifugation. A double-stranded plasmid was isolated from the cell pellet, and a single-stranded DNA from the supernatant. The overall cultures were stocked at -80 °C as a 15 % glycerol solution, the double-stranded plasmid and the single-stranded DNA were stocked at -20 °C as a TE solution, and the culture supernatant was stocked at 4 °C in a refrigerator.

### EXAMPLE 3

### Preparation of Human cDNA Bank

### (1) Size determination of human cDNA insert

The double-stranded plasmids isolated in section (5) of Example 2 were digested with EcoRI and NotI. The size of the resulting cDNA fragment was determined with 0.8 % agarose gel electrophoresis.

### (2) Determination of nucleotide sequence

The determination of the nucleotide sequence of a single-stranded cDNA prepared in section (5) of Example 2 was carried out using a DNA sequencer (purchased from Applied Biosystems). The sequencing reaction was performed by the dideoxy method using a fluorescence-labeled universal primer for M13 sequencing and Taq polymerase. This method could provide the nucleotide sequence of approximately 400 bp from 5′ end of cDNA. We found the 4 ∼ 10 tails of dG at the 5′ end of cDNA. The obtained nucleotide sequences were filed in the disk of computer as the "Homo-Protein cDNA database".

### (3) Analysis of nucleotide sequence

The filed nucleotide sequences were analyzed using the computer software including the genetic information analysis system GENIAS and the protein information analysis system PRINAS (both purchased from Mitsui Knowledge Industry). First, the homology search was carried out using each nucleotide sequence and the DNA database "GenBank^{R}". Then whether an initiation codon and an open reading frame exists in the amino acid sequence converted from the nucleotide sequence was investigated. If an open reading frame exists, the homology search of the amino acid sequence was carried out using the protein database "PRINAS".

Using above method a cDNA bank, that is, the group of cDNA vectors in which the size of cDNA insert, the nucleotide sequence from 5′ end, and the amino acid sequence encoded by the open reading frame were determined, was constructed. So far 20 % of analyzed clones had an open reading frame which sequence has a similarity with the amino acid sequence of a known protein in the database. Most of them are full-length clones. The obtained clones includes a wide variety of proteins such as metabolic enzymes, proteins regarding with DNA replication or protein synthesis, secreted proteins, and an extra-cellular matrix. Table 1 shows the examples of clones encoding proteins which have a similarity with a known protein.

### EXAMPLE 4

### Plaque Hybridization Using RNA Probe

### (1) Preparation of RNA probe

An RNA probe was prepared using a kit purchased from Stratagene. Briefly, after the double-stranded cDNA vector plasmid was cut with NotI, the product was reacted in the reaction mixture containing 50 µCi of [α-³²P] CTP and T7 RNA polymerase to synthesis an RNA probe corresponding to the sense strand of cDNA.

### (2) Plaque Hybridization

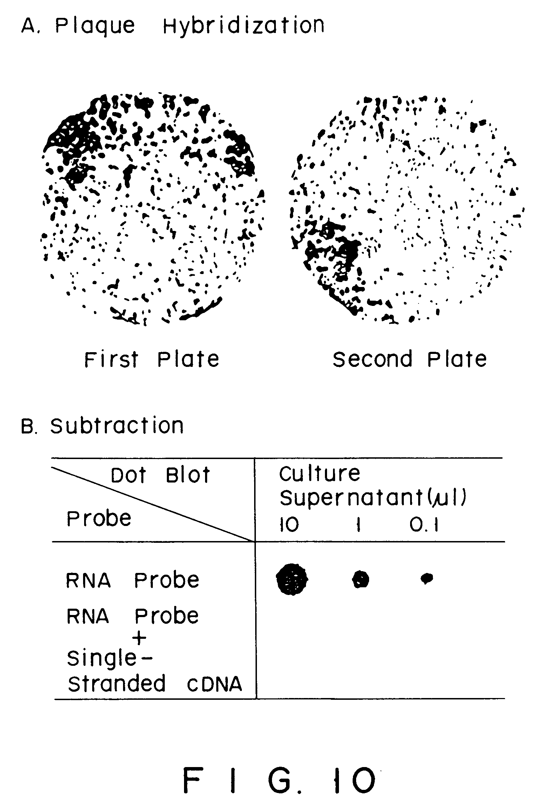

A cDNA plaque library was formed on agar plates using the method described in section (5) of Example 2. Dry nitrocellulose filters were placed on the surface of agar plates to transfer phages existing around the plaque. In the same way, two replica filters were prepared. The filters were air-dried, and baked at 80 °C for 3 hours. The filters were preincubated at 37 °C for 2 hours in the solution containing 50 % formamide, 5 x SSC, 10 x Denhardt's solution, and 0.1% SDS. After adding the RNA probe prepared in section (1), the incubation was sustained at 37 °C for 16 hours. The filters were washed with the solution containing 0.2 x SSC and 0.1% SDS at 65 °C for 1 hour, and air-dried. The resulting filters were subjected to autoradiography. Figure 10 (A) shows the result of a model experiment using a plasmid vector containing an actin cDNA. We can see positive signals where plaques exist.

### (3) Subtraction method

An RNA probe and a single-stranded DNA were prepared from the clone containing an actin cDNA, and prehybridized. The resulting mixture was used as a probe for hybridization with a filter spotted with phage containing a single-stranded actin cDNA. As shown in Figure 10 (B), we can see no positive signal on the autoradiogram. This means that a single-stranded DNA can inhibit completely the probe-function of a corresponding RNA by prehybridization, thus that the subtraction method shown in Figure 5 is possible.

### EXAMPLE 5

### Determination of Whole Nucleotide Sequence of cDNA Insert Using the Deletion Method

The plasmid pKATNF1 was isolated from the clone No.16 shown in Table 1 which contains the cDNA encoding TNF (Tumor Necrosis Factor). Fifteen µg of this plasmid was digested with 20 units of SphI and then 15 units of SnaBI. After treated with exonuclease III at 37 °C , the sampling was carried out at 1 minute interval. These samples were mixed, treated with mung-bean nuclease and Klenow fragment, and then self-ligated to obtain plasmids deleted from 5′ terminal of the cDNA insert. Above reaction was carried out using a kilo-deletion kit purchased from Takara Shuzo. The single-stranded DNA was prepared from each deleted clone using the method described in section (4) and (5) of Example 2, and used to determine its nucleotide sequence using the method described in section (2) of Example 3, where a reverse primer for M13 sequencing was used as a sequencing primer. As a result, plasmids lacking 150 bp, 300 bp, 600 bp, 850 bp, 1050 bp, and 1300 bp from 5′ terminal were obtained. The sequences from 5′ terminal of these clones were determined and overlapped to obtain the sequence covering a whole region of cDNA. The obtained sequence agreed completely with the reported sequence of TNF.

### EXAMPLE 6

### Expression of cDNA in Mammalian Cell

The pKATNF1 which sequence was determined in Example 5 was introduced into COS7 cells (obtained from ATCC), and its culture supernatant was tested whether it shows a TNF activity. The isolated plasmid pKATNF1 was transfected into 5 x 10⁶ cells of COS7 using an eletroporation method. After the trasnfected COS7 cells were inoculated in MEM medium for 3 days, the culture supernatant was subjected to a TNF assay using mouse L929 cells (obtained from ATCC). Consequently the supernatant showed a TNF activity of 2.9 x 10³ units/ml. This means that the SV40 promoter on the vector acts as expected.

**Table 1**

| Examples of clone containing in the cDNA bank. | |
|---|---|
| Clone number | Known protein showing similarity |
| 1 | HMG14 |
| 2 | HMG17 |
| 3 | Ribosomal protein S14 |
| 4 | Ribosomal protein S17 |
| 5 | Ribosomal phosphoprotein P1 |
| 6 | Elongation factor 1α |
| 7 | Initiation factor 2α |
| 8 | Glyceraldehyde-3-phosphate dehydrogenase |
| 9 | Pyruvate kinase |
| 10 | β-actin |
| 11 | Profilin |
| 12 | Heat shock protein 90kDa |
| 13 | Galactosidase A |
| 14 | Laminin binding protein |
| 15 | Plasminogen activator inhibitor 2 |
| 16 | TNF (Tumor Necrosis Factor) |
| 17 | Mitochondria cytochrome b |

## Claims

1. A cloning vector plasmid comprising, in the order mentioned from upstream to downstream region unless otherwise specified: (a) a promoter PR1 acting in mammalian cells; (b) in either order, a unique restriction enzyme site RE1 rarely existing in eukaryotic DNA and a promoter PR2 of RNA polymerase; (c) a unique restriction enzyme site RE2 generating 3'-protruding dN tail; (d) a unique arbitrary restriction enzyme site RE3; (e) a unique arbitrary restriction enzyme site RE4 generating a 3'-protruding terminus; (f) in either order, a unique restriction enzyme site RE5 rarely existing in eukaryotic DNA and a promoter PR3 of RNA polymerase; and (g) at least one kind of arbitrary restriction enzyme site RE6 generating a 3'-protruding terminus existing at an arbitrary position before the restriction site RE1; and further, at arbitrary positions other than the above-described restriction enzyme sites and promoters, an origin OR1 for replication in mammalian cells, an origin OR2 for replication of a single-stranded phage, an origin OR3 for replication in *E.coli*, and a selective marker.

2. The plasmid of Claim 1 wherein promoter PR1 is selected from an early or late promoter of SV40, a late promoter of adenovirus 2, and a 5' long terminal repeat (LTR) of retrovirus; restriction enzyme sites RE1 and RE5 are selected from NotI, SnaBI, SfiI, and SpeI; restriction enzyme site RE2 is selected from BstXI, SfiI, DraIII, and Bg1I; restriction enzyme site RE6 is a restriction site producing a 3'-protruding terminus and is different from RE1, RE2, RE3, RE4, and RE5; RNA polymerase promoters PR2 and PR3 are selected from promoters of T3 RNA polymerase, T7 RNA polymerase, and SP6 RNA polymerase; OR1 is an origin of SV40; OR2 is an origin of f1 phage or M13 phage; OR3 is an origin of plasmid pBR322 or of a plasmid of the pUC series.

3. The plasmid of claim 2 wherein RE6 contains more than two different restriction sites.

4. The plasmid of claim 1, which is a cloning vector plasmid pKA1 as disclosed in Example 1 of the present specification.

5. A vector-primer which is prepared by cutting said plasmid of any preceding claim at the restriction site RE4, adding thereto dT-tail by terminal deoxynucleotidyl transferase and then cutting the resultant product at the restriction site RE3.

6. The vector-primer of claim 5, wherein the plasmid of claim 4 is cut with restriction enzyme KpnI, the dT tail is added thereto with terminal deoxynucleotidyl transferase and then the resultant product is cut with restriction enzyme EcoRV.

7. A method for preparing a cDNA bank, said bank including cDNA vectors which each contain a sequence-determined cDNA insert and can be expressed in mammalian cells, which comprises synthesizing cDNAs from poly(A)⁺RNA isolated from cells using a vector-primer of claim 5 or claim 6, transforming *E.coli* with vectors containing said cDNAs, preparing a cDNA plaque library by infection of helper phage, and sequencing cDNA from the 5' terminal end using a single-stranded DNA isolated from a single-stranded phage plaque.

8. A method of preparing a library-specific cDNA bank, said bank including cDNA vectors which each contain a sequence-determined cDNA insert and can be expressed in mammalian cells, which comprises preparing two kinds of cDNA library LA and LB using the method of claim 7, preparing sense RNA probes from the library LA using RNA polymerase, preparing a group of antisense single-stranded cDNAs from the library LB, obtaining the library LA-specific cDNA clones by screening the library LA using probes which contain a hybridization mixture between RNA probes and said single-stranded cDNAs, and sequencing said library LA-specific cDNA from the 5' terminal.

## Patentansprüche

1. Kloniervektor-Plasmid, umfassend in der angegebenen Reihenfolge der Bereiche von stromauf nach stromab, falls nicht anders angegeben: (a) einen in Säugetierzellen wirksamen Promotor PR1; (b) in beliebiger Reihenfolge: eine einzige, in eukaryotischer DNA selten vorkommende Restriktions(enzym)stelle RE1 und einen RNA-Polymerase-Promotor PR2; (c) eine einzige, einen 3'-abstehenden dN-Schwanz erzeugende Restriktions(enzym)stelle RE2; (d) eine einzige, beliebige Restriktions(enzym)stelle RE3; (e) eine einzige, beliebige, einen 3'-abstehenden Terminus erzeugende Restriktions(enzym)stelle RE4; (f) in beliebiger Reihenfolge: eine einzige, in eukaryotischer DNA selten vorkommende Restriktions(enzym)stelle RE5 und einen RNA-Polymerase-Promotor PR3; und (g) zumindest eine Art einer beliebigen, einen 3'-abstehenden Terminus erzeugenden Restriktions(enzym)stelle RE6, der sich an beliebiger Position vor der Restriktionsstelle RE1 befinden kann; und weiters, an anderen beliebigen Positionen als jenen der zuvor beschriebenen Restriktions(enzym)stellen und Promotoren, einen Ursprung OR1 zur Replikation in Säugetierzellen, einen Ursprung OR2 zur Replikation eines einzelstrangigen Phagen, einen Ursprung OR3 zur Replikation in E.coli und einen selektiven Marker.

2. Plasmid nach Anspruch 1, worin der Promotor PR1 aus einem frühen oder späten Promotor von SV40, einem späten Promotor von Adenovirus 2 und einer Wiederholung des langen 5'-Terminus ("5'-long terminal repeat", LTR) von Retrovirus ausgewählt ist; die Restriktions(enzym)stellen RE1 und RE5 aus NotI, SnaBI, SfiI und SpeI ausgewählt sind; die Restriktions(enzym)stelle RE2 aus BstXI, SfiI, DraIII und BglI ausgewählt sind; die Restriktions(enzym)stelle RE6 eine einen 3'-abstehenden Terminus erzeugende Restriktionsstelle ist und sich von RE1, RE2, RE3, RE4 und RE5 unterscheidet; die RNA-Polymerase-Promotoren PR2 und PR3 aus T3-RNA-Polymerase-, T7-RNA-Polymerase- und SP6-RNA-Polymerase-Promotoren ausgewählt sind; OR1 ein Ursprung von SV40 ist; OR2 ein Ursprung von f1-Phagen oder M13-Phagen ist; und OR3 ein Ursprung von Plasmid pBR322 oder einem Plasmid der pUC-Reihe ist.

3. Plasmid nach Anspruch 2, worin RE6 mehr als zwei unterschiedliche Restriktionsstellen enthält.

4. Plasmid nach Anspruch 1, das ein Kloniervektor-Plasmid pKA1 ist, wie in Beispiel 1 der vorliegenden Beschreibung geoffenbart.

5. Vektor-Primer, der durch Spalten des Plasmids nach irgendeinem der vorangegangenen Ansprüche an der Restriktionsstelle RE4, daran Addieren eines dT-Schwanzes mittels terminaler Desoxynukleotidyl-Transferase und anschließendes Spalten des erhaltenen Produkts an der Restriktionsstelle RE3 hergestellt wird.

6. Vektor-Primer nach Anspruch 5, worin das Plasmid nach Anspruch 4 mit dem Restriktionsenzym Kpnl gespalten, der dT-Schwanz mittels terminaler Desoxynukleotidyl-Transferase daran addiert und das erhaltene Produkt dann mit dem Restriktionsenzym EcoRV gespalten wird.

7. Verfahren zur Herstellung einer cDNA-Bank, die cDNA-Vektoren umfaßt, die jeweils eine sequenzbestimmte cDNA-Einfügung enthalten und in Säugetierzellen exprimiert werden können, wobei das Verfahren umfaßt: die Synthese von cDNAs aus Poly(A)⁺RNA, die unter Verwendung eines Vektor-Primers nach Anspruch 5 oder 6 aus Zellen isoliert wurde, das Transformieren von E.coli mit diese cDNAs enthaltenden Vektoren, die Herstellung einer cDNA-Fleckensammlung durch Infektion von Helfer-Phagen, und das Sequenzieren der cDNA vom 5'-terminalen Ende weg unter Verwendung einer einzelstrangigen DNA, die aus einem einzelstrangigen Phagen-Fleck isoliert wurde.

8. Verfahren zur Herstellung einer sammlungsspezifischen cDNA-Bank, die cDNA-Vektoren umfaßt, die jeweils eine sequenzbestimmte cDNA-Einfügung enthalten und in Säugetierzellen exprimiert werden können, wobei das Verfahren umfaßt: die Herstellung von zwei Arten von cDNA-Sammlungen LA und LB unter Anwendung des Verfahrens nach Anspruch 7, die Herstellung von sense-RNA-Sonden aus der Sammlung LA mittels RNA-Polymerase, die Herstellung einer Gruppe von anti-sense, einzelstrangigen cDNAs aus der Sammlung LB, das Erhalten der LA-sammlungsspezifischen cDNA-Klone durch Screenen der Sammlung LA mittels Sonden, die ein Hybridisierungsgemisch zwischen RNA-Sonden und den einzelstrangigen cDNAs enthalten, und das Sequenzieren der LA-sammlungsspezifischen cDNA vom 5'-Terminus weg.

## Revendications

1. Vecteur plasmidique de clonage comprenant, dans l'ordre mentionné de la région en amont à en aval, sauf si mentionné autrement: (a) un promoteur PR1 agissant dans les cellules mammaliennes; (b) dans un ordre quelconque, un site d'enzyme de restriction unique RE1 existant rarement dans l'ADN eucaryote et un promoteur PR2 d'ARN polymérase; (c) un site d'enzyme de restriction unique RE2 générant une queue dN à extrémité 3' saillante; (d) un site d'enzyme de restriction unique arbitraire RE3; (e) un site d'enzyme de restriction unique arbitraire RE4 générant une extrémité 3' saillante; (f) dans un ordre quelconque, un site d'enzyme de restriction unique RE5 existant rarement dans l'ADN eucaryote et un promoteur PR3 d'ARN polymérase; et (g) au moins une sorte de site d'enzyme de restriction arbitraire RE6 générant une extrémité 3' saillante existant à une position arbitraire avant le site de restriction RE1; et de plus, aux positions arbitraires autres que les sites d'enzymes de restrictions et les promoteurs décrits ci-dessus, une origine OR1 pour une réplication dans des cellules mammaliennes, une origine OR2 pour une réplication d'un phage monocaténaire, une origine OR3 pour une réplication dans E. coli, et un marqueur sélectif.

2. Plasmide selon la revendication 1, dans lequel le promoteur PR1 est sélectionné parmi un promoteur précoce ou tardif de SV40, un promoteur tardif de l'adénovirus 2, et une répétition longue 5' terminale (LTR) d'un rétrovirus; les sites d'enzymes de restriction RE1 et RE5 sont sélectionnés parmi NotI, SnaBI, SfiI et SpeI; le site d'enzyme de restriction RE2 est sélectionné parmi BstXI, SfiI, DraIII et BglI; le site d'enzyme de restriction RE6 est un site de restriction produisant une extrémité 3' saillante et est différent de RE1, RE2, RE3, RE4 et RE5; les promoteurs d'ARN polymérase PR2 et FR3 sont sélectionnés parmi les promoteurs d'ARN polymérase T3, d'ARN polymérase T7 et d'ARN polymérase SF6; OR1 est une origine de SV40; OR2 est une origine du phage f1 ou du phage M13; OR3 est une origine du plasmide pBR322 ou d'un plasmide de la série des pUC.

3. Plasmide selon la revendication 2, dans lequel RE6 contient plus de deux sites de restriction différents.

4. Plasmide selon la revendication 1, qui est un vecteur plasmidique de clonage pKA1 comme décrit à l'Exemple 1 de la présente description.

5. Amorce de vecteur qui est préparée en coupant ledit plasmide de l'une quelconque des revendications précédentes au site de restriction RE4, en ajoutant à celui-ci une queue dT par une désoxynucléotidyl transférase terminale et ensuite en coupant le produit résultant au site de restriction RE3.

6. Amorce de vecteur selon la revendication 5, dans lequel le plasmide de la revendication 4 est coupé avec l'enzyme de restriction KpnI, la queue dT est ajoutée à celui-ci avec la désoxynucléotidyl transférase terminale et ensuite le produit résultant est coupé avec l'enzyme de restriction EcoRV.

7. Méthode pour préparer une banque d'ADNc, la banque incluant les vecteurs d'ADNc qui contiennent chacun un insert d'ADNc à séquence déterminée et qui peuvent être exprimés dans des cellules mammaliennes, qui comprend la synthèse d'ADNc à partir d'ARN poly(A)+ isolé de cellules en utilisant une amorce de vecteur selon la revendication 5 ou la revendication 6, la transformation d'E. coli avec les vecteurs contenant lesdits ADNc, la préparation d'une bibliothèque de plaques d'ADNc par infection d'un phage auxiliaire et le séquençage de l'ADNc à partir de l'extrémité 5' terminale en utilisant un ADN monocaténaire isolé d'une plaque de phages monocaténaire.

8. Méthode pour préparer une banque d'ADNc spécifique d'une bibliothèque, ladite banque incluant des vecteurs d'ADNc qui contiennent chacun un insert d'ADNc à séquence déterminée et qui peuvent être exprimés dans les cellules mammaliennes, qui comprend la préparation de deux sortes de bibliothèques d'ADNc LA et LB en utilisant la méthode de la revendication 7, la préparation de sondes d'ARN sens à partir de la bibliothèque LA en utilisant l'ARN polymérase, la préparation d'un groupe d'ADNc monocaténaires anti-sens à partir de la bibliothèque LB, l'obtention des clones d'ADNc spécifiques de la bibliothèque LA en triant la bibliothèque LA en utilisant des sondes qui contiennent un mélange d'hybridation entre les sondes d'ARN et lesdits ADNc monocaténaires, et le séquençage dudit ADNc spécifique de la bibliothèque LA à partir de l'extrémité 5'.
